# EUROPEAN PATENT SPECIFICATION

(11) **EP 3 422 853 B1**
(45) Date of publication and mention of the grant of the patent: **06.04.2022**
(21) Application number: 17760571.4
(22) Date of filing: 28.02.2017
(51) Int. Cl.: A01N 37/44, A01N 37/06, A01P 17/00

(54) **INSECT REPELLENT**
INSEKTENSCHUTZMITTEL
INSECTIFUGE

(30) Priority: 29.02.2016 US 201662301364 P
(43) Date of publication of application: 09.01.2019
(73) Proprietor: FMC Corporation, Philadelphia, PA 19104 (US)
(72) Inventor: ALBRIGHT, Robert, B., Lansdale PA 19446 (US); RICHMAN, Dina, L., Philadelphia PA 19134 (US); CALDWELL, Nathan, D., Morrisville PA 19067 (US); BLACK, Bruce, C., Yardley PA 19067 (US)
(74) Representative: Murgitroyd & Company
(86) International application number: PCT/US2017/019832
(87) International publication number: WO 2017/151549

(56) References cited:
- WO-A1-2015/009905
- WO-A1-2015/009911
- US-A- 5 296 226
- US-A1- 2015 025 141
- US-A1- 2015 250 166

## Description

### FIELD OF THE INVENTION

In one aspect, this invention relates to consumer care products comprising a pest repellent composition comprising a fatty acid and methyl anthranilate . In another aspect, this invention relates to a method of repelling pests employing such composition.

### BACKGROUND OF THE INVENTION

Insect and acarid pests can cause severe damage to crops and horticultural plants. Moreover insects and pests can carry agents that cause disease in both people and animals. For example, mosquitoes can carry viruses that cause encephalitis, and ticks can carry bacteria that cause Lyme disease. One method of controlling such pests involves the application of chemicals which repels such pests from a given environment. Thus, chemicals such as N,N-diethyl-m-toluamide (also known as DEET) is employed in repellents to protect individuals from mosquitoes, ticks and other similar pests. Other chemicals which affect insect behavior are used to attract or repel insects from a given environment in order to enhance the effectiveness of insecticides, either by attracting insects to the area where insecticides can be more effectively employed, or by repelling them from areas where insecticides are inefficient.

Among the compounds which have been employed to influence insect behavior so as to cause them to move to a different environment is methyl anthranilate. Thus, US Patent 6,958,146 (Askham et al), US Patent 7,867,479 (Dunham et al) and US Patent 8,092,790 (Dunham et al) all disclose the use of methyl anthranilate in the form of the commercial product BIRDSHIELD^{™} to induce insects to migrate from one environment to another (see, for example, Column 9, lines 26-33 of US 7,867,479). WO2015/009911A1 and WO2015/009905A1 are also useful.

Anthranilate esters such as methyl anthranilate have long been known to be useful as bird repellents. Thus, US Patent 2,967,128 (Kare) describes the use of such compounds to deter both domestic and wild birds from eating seeds, berries, grains, fruits and the like. Further, such compounds have found to be insect attractants -- for example, US Patent 5,296,226 (Askham) states (at Column 3, lines 20-23) that "insects are readily attracted to dimethyl and methyl anthanilate. Crops relatively free of insects were quickly reinfested after being treated with either material. The addition of soaps, however, reduced this problem" This finding is supported by the disclosures in US Patent 6,958,146 (Askham et al), US Patent 7,867,479 (Dunham et al) and US Patent 8,092,790 (Dunham et al) which show in Table 1 of such publications that sticky traps containing methyl anthranilate quickly became covered with hundreds of insects. The sole exception presented in these patents are house flies *(Musca domesticae)* which were repelled by the use of methyl anthranilate.

Although US Patents 6,958,146, 7,867,479 and 8,092,790 all describe BIRDSHIELD^{™} as being a mixture of methyl anthranilate with a fatty acid, it is noted that the label for such product indicates that it is covered by the claims of US Patent 5,296,226. This patent is directed to bird repellant compositions comprising an anionic surfactant consisting of an alkyl metal salt of a fatty acid; rather than a fatty acid in acid form. According to this publication, the addition of such fatty acid salts results in the formation of micelles of such anthranilate compounds, permitting a more even distribution of such compounds on the surface treated and enhancing their efficacy as bird repellents.

Given that compositions comprising an anthranilate ester and a fatty acid salt will attract most insects and will repel birds, it is completely unexpected that a composition comprising an anthranilate ester and a fatty acid in acid form will effectively repel many insect species as well as acarids, without repelling birds, making them useful for a number of repellent purposes including protecting bird feeders from unwanted insects.

Furthermore, many substances are applied topically to the skin or mucous membranes of humans or animals (hereafter "skin") in order to alter the subject's appearance, to protect the subject from the environment, or to produce a biological change in the skin or other tissue for therapeutic, preventive or cosmetic purposes. These substances may generically be termed "consumer care products" and include such topically applied substances as cosmetics, over-the-counter and prescription topical drugs, and a variety of other products such as soaps and detergents.

Consumer care products occur in a variety of forms, including solids, liquids, suspensions, semisolids (such as creams, gels, pastes or "sticks"), powders or finely dispersed liquids such as sprays or mists. Examples of consumer care products commonly classified as "cosmetics" include skin care products such as creams, lotions, moisturizers and "treatment cosmetics" such as exfoliants and/or skin cell renewal agents; fragrances such as perfumes and colognes, and deodorants; shaving-related products such as creams, "bracers" and aftershaves; depilatories and other hair removal products; skin cleansers, toners and astringents; pre-moistened wipes and washcloths; tanning lotions; bath products such as oils; eye care products such as eye lotions and makeup removers; foot care products such as powders and sprays; skin colorant and make-up products such as foundations, blushes, rouges, eye shadows and liners, lip colors and mascaras; lip balms and sticks; hair care and treatment products such as shampoos, conditioners, colorants, dyes, bleaches, straighteners and permanent wave products; baby products such as baby lotions, oils, shampoos, powders and wet wipes; feminine hygiene products such as deodorants and douches; skin or facial peels applied by dermatologists or cosmeticians; and others.

Examples of consumer care products commonly classified as "topical drugs" are many and varied, and include over-the-counter and/or prescription products such as antiperspirants, insect repellents, sunscreens and sunburn treatments, anti-acne agents, antibiotics, topical respiratory agents, ocular drugs such as eyedrops and saline solutions, therapeutic retinoids, anti-dandruff agents, external analgesics such as capsaicin products, topical contraceptives, topical drug delivery systems, gastrointestinal agents such as suppositories, enemas and hemorrhoid treatments, reproductive system agents such as vaginal treatments, oral treatments such as lozenges, and many other products with therapeutic or other effects. Other consumer care products include hand, facial and body soaps and detergents and other forms of skin cleansers, as well as household detergents and many other household products such as solvents, propellants, polishes, lubricants, adhesives, waxes and others which are either applied topically or are topically exposed to the body during normal use.

Applicants have recognized the need to develop consumer care products containing an anthranilate ester and a fatty acid salt that can effectively repel many insect species as well as acarids, without repelling birds, making them useful for a number of repellent purposes including protecting bird feeders from unwanted insects.

### SUMMARY OF THE INVENTION

In one aspect, the present invention is directed to a consumer care product comprising an insect- or acarid-repellant composition comprising:
(a) methyl anthranilate;
(b) a fatty acid selected from the group consisting of oleic acid, ricinoleic acid, linoleic acid, palmitic acid, stearic acid and any mixtures thereof;
(c) optionally at least one additional active ingredient;
(d) a pharmaceutically suitable topical carrier; and
(e) an additional compound selected from the group consisting of a surfactant, emulsifying agent, a pH modifier, a thickening agent, an anti-oxidant, a preservative, a skin conditioning agent, an emollient, a humectant, a silicone moiety, an anti-inflammatory, a sun blocking agent, a topical anesthetic, vitamins, an antipruritic, an antibiotic, antifungal, an antiseptic, a vasoconstrictor, and any mixtures thereof; wherein the weight ratio of methyl anthranilate to the fatty acid of part (b) is in the range of 5:1 to 4:1.

In another aspect this invention is directed to a non-therapeutic method of repelling insect and/or acarid pests by applying a composition as defined above to the surface of a human or an animal.

Optionally, the composition comprises an insect or acarid repellant described herein, wherein the fatty acid is oleic acid.

In certain embodiments, the emulsifying agent of part (e) is selected from the group consisting of polysaccharide ethers, polyglycosides, fatty acids, fatty alcohols, amine oxides, water-soluble cellulose, alkyl sulfonates, ethoxylated alkyl phenols, alkanolamides, betaines, zwitterionic surfactants, carboxylated alcohols, carboxylic acids, ethoxylated C12-C15 alcohols, polysorbates, behentrimonium methosulfate, isopropyl myristate and ethoxylated castor oil or any mixtures thereof.

In certain embodiments, the thickening agent is selected from the group consisting of carboxylic acid polymers, crosslinked acrylic acid with allyl ethers of sucrose, crosslinked polyacrylate polymers, polyacrylamide polymers, polysaccharides, gums and mixtures thereof.

In certain embodiments, the polysaccharides are selected from the group consisting of cellulose, carboxymethyl hydroxyethylcellulose, cellulose acetate propionate carboxylate, hydroxyethylcellulose, hydroxyethyl ethylcellulose, hydroxypropylcellulose, hydroxypropylmethylcellulose, methyl hydroxyethylcellulose, microcrystalline cellulose, sodium cellulose sulfate, chitosan and mixtures thereof.

Optionally, the pharmaceutically suitable topical carrier comprises water, glycerin, lecithin a fatty acid or any combinations thereof.

In certain embodiments, the sun blocking agent is selected from the group consisting of PABA, PABA esters, butyl PABA, ethyl PABA, ethyl dihydroxypropyl PABA, benzophenones, avobenzone, cinnamates, salicylates anthranilates, ethyl urocanate, homosalate, dibenzoylmethanes, octocrylene, methylbenzylidene camphor, kaolin, talc, petrolatum and metal oxides or any mixtures thereof.

In certain embodiments, , the subject is a human subject. In other embodiments, the subject is a livestock animal.

In certain embodiments, the composition is directly applied to the skin, the subject's apparel, and/or clothing.

The composition as defined in claim 1 may be deployed to a location whereby the insect is repelled when the insect comes into contact with the composition or vapors from the composition, wherein: the composition is deployed by; applying topically to an article of clothing or bedding; or laundering the article of clothing or bedding with a detergent or fabric softener or both that comprises the composition, or drying the article of clothing or bedding with a fabric softener that comprises the composition.

In further embodiments of this method, the composition is deployed by: applying the composition topically to an article of clothing of a human; or applying the composition topically to skin or hair of a human; or applying the composition topically to skin or fur of an animal; or laundering an article of clothing of a human with a detergent or fabric softener or both that comprises the composition; or drying an article of clothing of a human with a fabric softener that comprises the composition; or applying the composition to bedding, bed boards, bed slats, a mattress, box springs, furniture, carpeting, baseboards or flooring or a combination thereof; or spraying the composition on to the surface of bedding, bed boards, bed slats, a mattress, box springs, furniture or carpeting; or injecting the composition into the mattress, box springs, furniture or carpeting or a combination thereof; or deploying an absorbent substrate or gel containing the composition in the vicinity of bed boards, bed slats, a mattress, box springs, furniture or carpeting so that vapors from the composition come into contact with a surface of the bed boards, bed slats, a mattress, box springs, furniture or carpeting; or injecting the composition into a wall space.

In certain embodiments, the personal care or cosmetic composition is formulated as a product selected from among insect repellents, skin care products, hair care products, and cleansing products.

In other embodiments, the composition comprising an insect or acarid repellant provided herein is formulated as a household composition.

### DETAILED DESCRIPTION OF THE INVENTION

The present invention is set out in the claims.

The fatty acids are employed as component (b) are oleic acid, ricinoleic acid, linoleic acid palmitic acid and stearic acid; with oleic acid being particularly preferred.

Illustrative of further components which may be included in the compositions of this invention are antioxidant agents which serve to substantially prolong the desirable action of the fatty acid. Such antioxidant agent(s) protect the chemical and physical integrity of the fatty acid against reaction with oxygen and air pollution alone or in the presence of light. There exists an abundance of suitable antioxidants consisting of commercial and specialty chemicals and their combinations, mixtures and proprietary compositions that are well known to those educated in the art.

In certain embodiments, one particular antioxidant agent is selected from a group that includes, but is not limited to, ascorbic acid palmitate, ascorbyl palmitate, butylated p-cresol, tert-butylhydroquinone, butylated hydroquinone monomethyl ether, butylhydroxy-anisole, butylhydroxytoluene, propyl gallate and a tocopherol and combinations thereof.. The amount of the antioxidant is preferably from 0.001 - 0.1 % by weight of the total composition.

The composition comprising an insect or acrid repellant provided herein may further comprises at least one additional active ingredient selected from the group consisting of pharmaceutically active agents, insecticides, pesticides, fungicides, herbicides, fertilizers, repellant and combinations thereof. Suitable additional active ingredients for the insect or acrid repellant provided herein include the following:

Insecticides: A1) the class of carbamates consisting of aldicarb, alanycarb, benfuracarb, carbaryl, carbofuran, carbosulfan, methiocarb, methomyl, oxamyl, pirimicarb, propoxur and thiodicarb; A2) the class of organophosphates consisting of acephate, azinphos-ethyl, azinphos-methyl, chlorfenvinphos, chlorpyrifos, chlorpyrifos-methyl, demeton-S-methyl, diazinon, dichlorvos/DDVP, dicrotophos, dimethoate, disulfoton, ethion, fenitrothion, fenthion, isoxathion, malathion, methamidaphos, methidathion, mevinphos, monocrotophos, oxymethoate, oxydemeton-methyl, parathion, parathion-methyl, phenthoate, phorate, phosalone, phosmet, phosphamidon, pirimiphos-methyl, quinalphos, terbufos, tetrachlorvinphos, triazophos and trichlorfon; A3) the class of cyclodiene organochlorine compounds such as endosulfan; A4) the class of fiproles consisting of ethiprole, fipronil, pyrafluprole and pyriprole; A5) the class of neonicotinoids consisting of acetamiprid, chlothianidin, dinotefuran, imidacloprid, nitenpyram, thiacloprid and thiamethoxam; A6) the class of spinosyns such as spinosad and spinetoram; A7) chloride channel activators from the class of mectins consisting of abamectin, emamectin benzoate, ivermectin, lepimectin and milbemectin; A8) juvenile hormone mimics such as hydroprene, kinoprene, methoprene, fenoxycarb and pyriproxyfen; A9) selective homopteran feeding blockers such as pymetrozine, flonicamid and pyrifluquinazon; A10) mite growth inhibitors such as clofentezine, hexythiazox and etoxazole; A11) inhibitors of mitochondrial ATP synthase such as diafenthiuron, fenbutatin oxide and propargite; uncouplers of oxidative phosphorylation such as chlorfenapyr; A12) nicotinic acetylcholine receptor channel blockers such as bensultap, cartap hydrochloride, thiocyclam and thiosultap sodium; A13) inhibitors of the chitin biosynthesis type 0 from the benzoylurea class consisting of bistrifluron, diflubenzuron, flufenoxuron, hexaflumuron, lufenuron, novaluron and teflubenzuron; A14) inhibitors of the chitin biosynthesis type 1 such as buprofezin; A15) moulting disruptors such as cyromazine; A16) ecdyson receptor agonists such as methoxyfenozide, tebufenozide, halofenozide and chromafenozide; A17) octopamin receptor agonists such as amitraz; A18) mitochondrial complex electron transport inhibitors pyridaben, tebufenpyrad, tolfenpyrad, flufenerim, cyenopyrafen, cyflumetofen, hydramethylnon, acequinocyl or fluacrypyrim; A19) voltage-dependent sodium channel blockers such as indoxacarb and metaflumizone; A20) inhibitors of the lipid synthesis such as spirodiclofen, spiromesifen and spirotetramat; A21) ryanodine receptor-modulators from the class of diamides consisting of flubendiamide, the phthalamide compounds (R)-3-Chlor-N1-{2-methyl-4-[1,2,2,2-tetrafluor-1-(trifluormethyl)ethyl]phenyl}-N2-(1-methyl-2-methylsulfonylethyl)phthalamid and (S)-3-Chlor-N1-{2-methyl-4-[1,2,2,2-tetrafluor-1-(trifluormethyl)ethyl]phenyl}-N2-(1-methyl-2-methylsulfonylethyl)phthalamid, chloranthraniliprole and cyanthraniliprole; A22) compounds of unknown or uncertain mode of action such as azadirachtin, amidoflumet, bifenazate, fluensulfone, piperonyl butoxide, pyridalyl, sulfoxaflor; or A23) sodium channel modulators from the class of pyrethroids consisting of acrinathrin, allethrin, bifenthrin, cyfluthrin, lambdacyhalothrin, cypermethrin, alpha-cypermethrin, beta-cypermethrin, zetacypermethrin, deltamethrin, esfenvalerate, etofenprox, fenpropathrin, fenvalerate, flucythrinate, tau-fluvalinate, permethrin, silafluofen and tralomethrin.

Fungicides: B1) azoles selected from the group consisting of bitertanol, bromuconazole, cyproconazole, difenoconazole, diniconazole, enilconazole, epoxiconazole, fluquinconazole, fenbuconazole, flusilazole, flutriafol, hexaconazole, imibenconazole, ipconazole, metconazole, myclobutanil, penconazole, propiconazole, prothioconazole, simeconazole, triadimefon, triadimenol, tebuconazole, tetraconazole, triticonazole, prochloraz, pefurazoate, imazalil, triflumizole, cyazofamid, benomyl, carbendazim, thia- bendazole, fuberidazole, ethaboxam, etridiazole and hymexazole, azaconazole, diniconazole-M, oxpoconazol, paclobutrazol, uniconazol, 1-(4-chlorophenyl)-2-([1,2,4]triazol-1-yl)-cycloheptanol and imazalilsulfphate; B2) strobilurins selected from the group consisting of azoxystrobin, dimoxystrobin, enestroburin, fluoxastrobin, kresoxim-methyl, methominostrobin, orysastrobin, picoxystrobin, pyraclostrobin, trifloxystrobin, enestroburin, methyl (2-chloro-5-[1-(3-methylbenzyloxyimino)ethyl]benzyl)carbamate, methyl (2-chloro-5-[1-(6-methylpyridin-2-ylmethoxyimino)ethyl]benzyl)carbamate and methyl 2-(ortho-(2,5-dimethylphenyloxymethylene)- phenyl)-3-methoxyacrylate, 2-(2-(6-(3-chloro-2-methyl-phenoxy)-5-fluoro-pyrimidin-4-yloxy)-phenyl)-2-methoxyimino-N-methyl-acetamide and 3-methoxy-2-(2-(N-(4-methoxyphenyl)cyclopropanecarboximidoylsulfanylmethyl)-phenyl)-acrylic acid methyl ester; B3) carboxamides selected from the group consisting of carboxin, benalaxyl, benalaxyl-M, fenhexamid, flutolanil, furametpyr, mepronil, metalaxyl, mefenoxam, ofurace, oxadixyl, oxycarboxin, penthiopyrad, isopyrazam, thifluzamide, tiadinil, 3,4-dichloro-N-(2-cyanophenyl)isothiazole-5-carboxamide, dimethomorph, flumorph, flumetover, fluopicolide (picobenzamid), zoxamide, carpropamid, diclocymet, mandipropamid, N-(2- (4-[3-(4-chlorophenyl)prop-2-ynyloxy]-3-methoxyphenyl)ethyl)-2- methanesulfonyl-amino-3-methylbutyramide, N-(2-(4-[3-(4-chlorophenyl)prop-2-ynyloxy]-3-methoxy-phenyl)ethyl)-2-ethanesulfonylamino-3-methylbutyramide, methyl 3-(4-chlorophenyl)-3-(2-isopropoxycarbonyl-amino-3-methyl-butyrylamino)propionate, N-(4'-bromobiphenyl-2-yl)-4-difluoromethylmethylthiazole-.-carboxamide, N-(4'-trifluoromethyl- biphenyl-2-yl)-4-difluoromethyl-2-methylthiazole-5-carboxamide, N-(4'-chloro-3'-fluorobiphenyl-2-yl)-4-difluoromethyl-2-methyl-thiazole-5-carboxamide, N-(3,4'-dichloro-4-fluorobiphenyl-2-yl)-3-difluoro-methyl-1-methyl-pyrazole-4-carboxamide, N-(3',4'-dichloro-5-fluorobiphenyl-2-yl)-3-difluoromethyl-1-methylpyrazole-4-carboxamide, N-(2-cyano-phenyl)- 3,4-dichloroisothiazole-5-carboxamide, 2-amino-4-methylthiazole-5-carboxanilide, 2-chloro-N-(1,1,3-trimethyl-indan-4-yl)-nicotinamide, N-(2-(1,3-dimethylbutyl)-phenyl)-1,3-dimethyl-5-fluoro-1H-pyrazole-4-carboxamide, N-(4'-chloro-3',5-difluoro-biphenyl-2-yl)-3-difluoromethyl-1-methyl-I H-pyrazole-4-carboxamide, N-(4'-chloro-3',5-difluoro-biphenyl- 2-yl)-3-trifluoromethyl-1-methyl-1H-pyrazole-4-carboxamide, N-(3',4'- dichloro-5-fluoro-biphenyl-2-yl)-3-trifluoromethyl-1-methyl-1H-pyrazole-4-carboxamide, N-(3',5-difluoro-4'-methylbiphenyl-2-yl)-3-difluoromethyl-1-methyl-1H-pyrazole-4-carboxamide, N-(3',5-difluoro-4'-methyl-biphenyl-2-yl)-3-trifluoromethyl-1-methyl-1H-pyrazole-4-carboxamide, N-(cis-2-bicyclopropyl-2-yl-phenyl)-3-difluoromethyl-1-methyl-1H-pyrazole-4-carboxamide, N-(trans-2-bicyclopropyl-2-yl-phenyl)-3-difluoro-methyl-1-methyl-1H-pyrazole-4-carboxamide, fluopyram, N-(3-ethyl-3,5-5- trimethylcyclohexyl)-3-formylamino-2-hydroxy-benzamide, oxytetracyclin, silthiofam, N-(6-methoxy-pyridin-3-yl)cyclopropanecarboxamide, 2-iodo-N-phenyl-benzamide, N-(2-bicyclo-propyl-2-yl-phenyl)-3-difluormethyl-1-methylpyrazol-4-ylcarboxamide, N-(3',4',5'-trifluorobiphenyl-2-yl)-1,3-dimethylpyrazol-4-ylcarboxamide, N-(3',4',5'-trifluorobiphenyl-2-yl)-1,3-dimethyl-5-fluoropyrazol-4-ylcarboxamide, N-(3',4',5'-trifluorobiphenyl-2-yl)-5-chloro-1,3-dimethyl-pyrazol-4-ylcarboxamide, N-(3',4',5'-trifluorobiphenyl-2-yl)-3-fluoromethyl-1-methylpyrazol-4-ylcarboxamide, N-(3',4',5'-trifluorobiphenyl-2-yl)-3-(chlorofluoromethyl)-1-methylpyrazol-4-ylcarboxamide,N-(3',4',5'-trifluorobiphenyl-2-yl)-3-difluoromethyl-1-methylpyrazol-4-ylcarboxamide, N-(3',4', 5'-trifluorobiphenyl-2-yl)-3-difluoromethyl-5-fluoro-1-methylpyrazol-4-ylcarboxamide, N-(3',4',5'-trifluorobiphenyl-2-yl)-5-chloro-3-difluoromethyl-1-methylpyrazol-4-ylcarboxamide, N- (3', 4', 5'-trifluorobiphenyl-2-yl)-3-(chlorodifluoromethyl)-1-methylpyrazol-4-ylcarboxamide, N-(3',4',5'-trifluorobiphenyl-2-yl)-1-methyl-3-trifluoromethylpyrazol-4-ylcarboxamide, N-(3',4',5'-trifluorobiphenyl-2-yl)- 5-fluoro-1-methyl-3-trifluoromethylpyrazol-4-ylcarboxamide, N-(3',4',5'-trifluorobiphenyl-2-yl)-5-chloro-1-methyl-3-trifluoromethylpyrazol-4-ylcarboxamide, N-(2',4',5'-trifluorobiphenyl-2-yl)-1,3-dimethylpyrazol-4-ylcarboxamide, N-(2',4',5'-trifluorobiphenyl-2-yl)-1,3-dimethyl-5-fluoropyrazol-4-ylcarboxamide, N-(2',4',5'- trifluorobiphenyl-2-yl)-5-chloro-1,3-dimethylpyrazol-4-ylcarboxamide, N- (2',4',5'-trifluorobiphenyl-2-yl)-3-fluoromethyl-1-methylpyrazol-4-ylcarboxamide, N-(2',4',5'-trifluorobiphenyl-2-yl)-3-(chlorofluoromethyl)-1-methylpyrazol-4-ylcarboxamide,N-(2',4',5'-trifluorobiphenyl-2-yl)-3-difluoromethyl-1-methylpyrazol-4-ylcarboxamide, N-(2',4',5'-trifluorobiphenyl-2-yl)-3-difluoromethyl-5- fluoro-1-methylpyrazol-4-ylcarboxamide, N-(2',4',5'-trifluorobiphenyl-2- yl)-5-chloro-3-difluoromethyl-1-methylpyrazol-4-ylcarboxamide, N-(2',4',5'-trifluorobiphenyl-2-yl)-3-(chlorodifluoromethyl)-1-methylpyrazol-4-ylcarboxamide, N-(2',4',5'-trifluorobiphenyl-2-yl)-1-methyl-3-trifluoromethylpyrazol-4-ylcarboxamide, N-(2',4',5'-trifluorobiphenyl-2-yl)-5-fluoro-1-methyl-3-trifluoromethylpyrazol-4-ylcarboxamide, N-(2',4',5'-trifluorobiphenyl-2-yl)-5-chloro-1-methyl-3-trifluoromethylpyrazol-4-ylcarboxamide, N-(3',4'-dichloro-3-fluorobiphenyl-2-yl)-1-methyl-3-trifluoromethyl-1H-pyrazole-4-carboxamide, N-(3',4'-dichloro-3-fluorobiphenyl-2-yl)-1-methyl-3-difluoromethyl-1H-pyrazole-4-carboxamide, N-(3',4'-difluoro-3-fluorobiphenyl-2-yl)-1-methyl-3-trifluoromethyl-1H-pyrazole-4-carboxamide, N-(3',4'-difluoro-3-fluorobiphenyl-2-yl)-1-methyl-S-difluoromethyl-1H-pyrazole-4-carboxamide, N-(3'-chloro-4'-fluoro-3-fluorobiphenyl-2-yl)-1-methyl-3-difluoromethyl-1H-pyrazole-4-carboxamide, N-(3',4'-dichloro-4-fluorobiphenyl-2-yl)-1-methyl-3-trifluoromethyl-1H-pyrazole-4-carboxamide, N-(3',4'-difluoro-4-fluorobiphenyl-2-yl)-1-methyl-S-trifluoromethyl-1H-pyrazole-4-carboxamide, N-(3',4'-dichloro-4-fluorobiphenyl-2-yl)-1-methyl-3-difluoromethyl-1H-pyrazole-4-carboxamide, N-(3',4'-difluoro-4-fluorobiphenyl-2-yl)-1-methyl-3 - difluoromethyl-1H- pyrazole-4-carboxamide, N-(3'-chloro-4'-fluoro-4-fluorobiphenyl-2-yl)-1-methyl-S-difluoromethyl-1H-pyrazole-4-carboxamide, N-(3',4'-dichloro-5- fluorobiphenyl-2-yl)-1-methyl-3-trifluoromethyl-1H-pyrazole-4-carboxamide, N-(3',4'-difluoro-5-fluorobiphenyl-2-yl)-1-methyl-3-trifluoromethyl-1H-pyrazole-4-carboxamide, N-(3',4'-dichloro-5-fluorobiphenyl-2-yl)-1-methyl-S-difluoromethyl-1H-pyrazole-4-carboxamide, N-(3',4'-difluoro-5-fluorobiphenyl-2-yl)-1-methyl-3-difluoromethyl-1H-pyrazole-4-carboxamide, N-(3',4'-dichloro-5-fluorobiphenyl-2-yl)-1,3-dimethyl-1H-pyrazole-4-carboxamide, N-(3'-chloro-4'-fluoro-5-fluorobiphenyl-2-yl)-1-methyl-3-difluoromethyl-1H-pyrazole-4-carboxamide, N-(4'-fluoro-4-fluorobiphenyl-2-yl)-1-methyl-3-trifluoromethyl-1H-pyrazole-4-carboxamide, N-(4'-fluoro- 5-fluorobiphenyl-2-yl)-1-methyl-3-trifluoromethyl-1H-pyrazole-4-carboxamide,N-(4'-chloro-5-fluorobiphenyl-2-yl)-1-methyl-3-trifluoromethyl-1H- pyrazole-4-carboxamide, N-(4'-methyl-5-fluorobiphenyl-2-yl)-1-methyl-3-trifluoromethyl-1H-pyrazole-4-carboxamide, N-(4'-fluoro-5- fluorobiphenyl-2-yl)-1,3-dimethyl-1H-pyrazole-4-carboxamide, N-(4'-chloro-5-fluorobiphenyl-2-yl)-1,3-dimethyl-1H-pyrazole-4-carboxamide, N-(4'-methyl-5-fluorobiphenyl-2-yl)-1,3-dimethyl-1H-pyrazole-4-carboxamide, N-(4'-fluoro-6-fluorobiphenyl-2-yl)-1-methyl-3 -trifluoromethyl-1H- pyrazole-4-carboxamide, N-(4'-chloro-6-fluorobiphenyl-2-yl)-1-methyl-3-trifluoromethyl-1H-pyrazole-4-carboxamide, N-[2-(1,1,2,3,3,3- hexafluoropropoxy)-phenyl]-3-difluoromethyl-1-methyl-1H-pyrazole-4-carboxamide, N-[4'-(trifluoromethylthio)-biphenyl-2-yl]-3-difluoromethyl-1-methyl-1H-pyrazole-4-carboxamide and N-[4'-(trifluoromethylthio)-biphenyl-2-yl]-1-methyl-3-trifluoromethyl-1-methyl-1H-pyrazole-4-carboxamide; B4) heterocyclic compounds selected from the group consisting of fluazinam, pyrifenox, bupirimate, cyprodinil, fenarimol, ferimzone, mepanipyrim, nuarimol, pyrimethanil, triforine, fenpiclonil, fludioxonil, aldimorph, dodemorph, fenpropimorph, tridemorph, fenpropidin, iprodione, procymidone, vinclozolin, famoxadone, fenamidone, octhilinone, proben- azole, 5-chloro-7-(4-methyl-piperidin-1-yl)-6-(2,4,6-trifluorophenyl)-[1,2,4]triazolo[1,5-a]pyrimidine, anilazine, diclomezine, pyroquilon, proquinazid, tricyclazole, 2-butoxy-6-iodo-3-propylchromen-4-one, acibenzolar-S-methyl, captafol, captan, dazomet, folpet, fenoxanil, quinoxyfen, N,N-dimethyl-3 -(3 -bromo-6-fluoro-2-methylindole-1-sulfonyl)- [1,2,4]triazole-1-sulfonamide, 5-ethyl-6-octyl-[1,2,4]triazolo[1,5-a]pyrimidin-2,7-diamine, 2,3,5,6-tetrachloro-4-methanesulfonyl-pyridine, 3,4,5-trichloro-pyridine-2,6-di-carbonitrile, N-(1-(5-bromo-3-chloro-pyridin-2-yl)-ethyl)-2,4-dichloro-nicotinamide, N-((5-bromo-3-chloro pyridin-2-yl)-methyl)-2,4-dichloro-nicotinamide, diflumetorim, nitrapyrin, dodemorphacetate, fluoroimid, blasticidin-S, chinomethionat, debacarb, difenzoquat, difenzoquat-methylsulphate, oxolinic acid and piperalin; B5) carbamates selected from the group consisting of mancozeb, maneb, metam, methasulphocarb, metiram, ferbam, propineb, thiram, zineb, ziram, diethofencarb, iprovalicarb, benthiavalicarb, propamocarb, propamocarb hydrochlorid, 4-fluorophenyl N-(1-(1-(4-cyanophenyl)-ethanesulfonyl)but-2-yl)carbamate, methyl 3-(4-chloro-phenyl)-3-(2- isopropoxycarbonylamino-3-methyl-butyrylamino)propanoate; or B6) other fungicides selected from the group consisting of guanidine, dodine, dodine free base, iminoctadine, guazatine, antibiotics: kasugamycin, streptomycin, polyoxin, validamycin A, nitrophenyl derivatives: binapacryl, dinocap, dinobuton, sulfur-containing heterocyclyl compounds: dithianon, isoprothiolane, organometallic compounds: fentin salts, organophosphorus compounds: edifenphos, iprobenfos, fosetyl, fosetyl-aluminum, phosphorous acid and its salts, pyrazophos, tolclofos- methyl, organochlorine compounds: dichlofluanid, flusulfamide, hexachloro- benzene, phthalide, pencycuron, quintozene, thiophanatemethyl, tolylfluanid, others: cyflufenamid, cymoxanil, dimethirimol, ethirimol, furalaxyl, metrafenone and spiroxamine, guazatine-acetate, iminoctadine-triacetate, iminoctadine-tris(albesilate), kasugamycin hydrochloride hydrate, dichlorophen, pentachlorophenol and its salts, N-(4- chloro-2-nitro-phenyl)-N-ethyl-4-methyl-benzenesulfonamide, dicloran, nitrothal-isopropyl, tecnazen, biphenyl, bronopol, diphenylamine,mildiomycin, oxincopper, prohexadione calcium, N-(cyclopropylmethoxyimino-(6-difluoromethoxy-2,3-difluoro-phenyl)- methyl)-2-phenyl acetamide, N'-(4-(4-chloro-3-trifluoromethyl-phenoxy)-2,5-dimethyl-phenyl)-N-ethyl-N-methyl formamidine, N'-(4-(4-fluoro-3-trifluoromethyl-phenoxy)-2,5-dimethyl-phenyl)-N-ethyl-N-methyl formamidine, N'-(2-methyl-5-trifluormethyl-4-(3-trimethylsilanyl-propoxy)-phenyl)-N-ethyl-N-methylformamidine and N'-(5-difluormethyl-2-methyl-4-(3-trimethylsilanyl-propoxy)-phenyl)-N-ethyl-N-methyl formamidine.

Herbicides: C1) acetyl-CoA carboxylase inhibitors (ACC), for example cyclohexenone oxime ethers, such as alloxydim, clethodim, cloproxydim, cycloxydim, sethoxydim, tralkoxydim, butroxydim, clefoxydim or tepraloxydim; phenoxyphenoxypropionic esters, such as clodinafop-propargyl, cyhalofop-butyl, diclofop-methyl, fenoxaprop-ethyl, fenoxaprop-P-ethyl, fenthiapropethyl, fluazifopbutyl, fluazifop-P-butyl, haloxyfop-ethoxyethyl, haloxyfop-methyl, haloxyfop-P-methyl, isoxapyrifop, propaquizafop, quizalofop-ethyl, quizalofop-P-ethyl or quizalofop-tefuryl; or arylaminopropionic acids, such as flamprop-methyl or flamprop-isopropyl; C2) acetolactate synthase inhibitors (ALS), for example imidazolinones, such as imazapyr, imazaquin, imazamethabenz-methyl (imazame), imazamox, imazapic or imazethapyr; pyrimidyl ethers, such as pyrithiobac-acid, pyrithiobac-sodium, bispyribac-sodium, KIH-6127 or pyribenzoxym; sulfonamides, such as florasulam, flumetsulam or metosulam; or sulfonylureas, such as amidosulfuron, azimsulfuron, bensulfuron-methyl, chlorimuron-ethyl, chlorsulfuron, cinosulfuron, cyclosulfamuron, ethametsulfuron-methyl, ethoxysulfuron, flazasulfuron, halosulfuron-methyl, imazosulfuron, metsulfuron-methyl, nicosulfuron, primisulfuron-methyl, prosulfuron, pyrazosulfuron-ethyl, rimsulfuron, sulfometuronmethyl, thifensulfuron-methyl, triasulfuron, tribenuron-methyl, triflusulfuron-methyl, tritosulfuron, sulfosulfuron, foramsulfuron or iodosulfuron; C3) amides, for example allidochlor (CDAA), benzoylprop-ethyl, bromobutide, chlorthiamid. diphenamid, etobenzanid, fluthiamide, fosamin or monalide; C4) auxin herbicides, for example pyridinecarboxylic acids, such as clopyralid or picloram; or 2,4-D or benazolin; C5) auxin transport inhibitors, for example naptalame or diflufenzopyr; C6) carotenoid biosynthesis inhibitors, for example benzofenap, clomazone, diflufenican, fluorochloridone, fluridone, pyrazolynate, pyrazoxyfen, isoxaflutole, isoxachlortole, mesotrione, sulcotrione (chlormesulone), ketospiradox, flurtamone, norflurazon or amitrol; C7) enolpyruvylshikimate-3-phosphate synthase inhibitors (EPSPS), for example glyphosate or sulfosate; C8) glutamine synthetase inhibitors, for example bilanafos or glufosinate-ammonium; C9) lipid biosynthesis inhibitors, for example anilides, such as anilofos or mefenacet; chloroacetanilides, such as dimethenamid, S-dimethenamid, acetochlor, alachlor, butachlor, butenachlor, diethatyl-ethyl, dimethachlor, metazachlor, metolachlor, S-metolachlor, pretilachlor, propachlor, prynachlor, terbuchlor, thenylchlor or xylachlor; thioureas, such as butylate, cycloate, di-allate, dimepiperate, EPTC. esprocarb, molinate, pebulate, prosulfocarb, thiobencarb (benthiocarb), tri-allate or vemolate; or benfuresate or perfluidone; C10) mitosis inhibitors, for example carbamates, such as asulam, carbetamid, chlorpropham, orbencarb, propyzamid, propham or tiocarbazil; dinitroanilines, such as benefin, butralin, dinitramin, ethalfluralin, fluchloralin, oryzalin, pendimethalin, prodiamine or trifluralin; pyridines, such as dithiopyr or thiazopyr; or butamifos, chlorthal-dimethyl (DCPA) or maleic hydrazide; C11) protoporphyrinogen IX oxidase inhibitors, for example diphenyl ethers, such as acifluorfen, acifluorfensodium, aclonifen, bifenox, chlomitrofen (CNP), ethoxyfen, fluorodifen, fluoroglycofen-ethyl, fomesafen, furyloxyfen, lactofen, nitrofen, nitrofluorfen or oxyfluorfen; oxadiazoles, such as oxadiargyl or oxadiazon; cyclic imides, such as azafenidin, butafenacil, carfentrazone, carfentrazone-ethyl, cinidon-ethyl, flumiclorac-pentyl, flumioxazin, flumipropyn, flupropacil, fluthiacet-methyl, sulfentrazone or thidiazimin; or pyrazoles, such as ET-751, JV 485 or nipyraclofen; C12) photosynthesis inhibitors, for example propanil, pyridate or pyridafol; benzothiadiazinones, such as bentazone; dinitrophenols, for example bromofenoxim, dinoseb, dinoseb-acetate, dinoterb or DNOC; dipyridylenes, such as cyperquatchloride, difenzoquat-methylsulfate, diquat or paraquat-dichloride; ureas, such as chlorbromuron, chlorotoluron, difenoxuron, dimefuron, diuron, ethidimuron, fenuron, fluometuron, isoproturon, isouron, linuron, methabenzthiazuron, methazole, metobenzuron, metoxuron, monolinuron, neburon, siduron or tebuthiuron; phenols, such as bromoxynil or ioxynil; chloridazon; triazines, such as ametryn, atrazine, cyanazine, desmein, dimethamethryn, hexazinone, prometon, prometryn, propazine, simazine, simetryn, terbumeton, terbutryn, terbutylazine or trietazine; triazinones, such as metamitron; uracils, such as bromacil, lenacil or terbacil; or biscarbamates, such as desmedipham or phenmedipham; C13) synergists, for example oxiranes, such as tridiphane; C14) CIS cell wall synthesis inhibitors, for example isoxaben or dichlobenil; C16) various other herbicides, for example dichloropropionic acids, such as dalapon; dihydrobenzofurans, such as ethofumesate; phenylacetic acids, such as chlorfenac (fenac); or aziprotryn, barban, bensulide, benzthiazuron, benzofluor, buminafos, buthidazole, buturon, cafenstrole, chlorbufam, chlorfenprop-methyl, chloroxuron, cinmethylin, cumyluron, cycluron, cyprazine, cyprazole, dibenzyluron, dipropetryn, dymron, eglinazin-ethyl, endothall, ethiozin, flucabazone, fluorbentranil, flupoxam, isocarbamid, isopropalin, karbutilate, mefluidide, monuron, napropamide, napropanilide, nitralin, oxaciclomefone, phenisopham, piperophos, procyazine, profluralin, pyributicarb, secbumeton, sulfallate (CDEC), terbucarb, triaziflam, triazofenamid or trimeturon; or their environmentally compatible salts.

Repellants include, but are not limited to, dimethyl anthranilate, methyl anthranilate, ethyl anthranilate, phenylethyl anthranilate and menthyl anthranilate and their environmentally compatible salts, N, N-diethyl-meta-toluamide (DEET), picaridin, oil of lemon eucalyptus, citronella, 2-undecanone, ethyl butyl acetyl aminoproprionate (IR3535), A13-37220, tansy essential oil, amitraz, permethrin, dimethyl phthalate, R-11, and ethyl hexane diol.

In the present invention, consumer care products are described for repelling insects and/or acarid pests. Consumer care products include, but are not limited to, topical compositions for application to the skin, clothing or other material to repel insect and/or acarid pests containing an insect or cararid repellant component such as a pheromone, an essence, an anthranilate ester, O-aminoacetphones, methylbenzamides such as N,N di-ethyl-3-methylbenzamide or N,N diethyl-m-toluamide (DEET), repellant oils such as tee tree oil, lemongrass oil, lemongrass oil, citronella oil, eucalyptus oil, glavender oil, geranium oil, clove oil, cajeput oil and lemon balm oil, and a fatty acid or any mixtures thereof. In certain embodiments of the invention, the product is a composition in liquid or semi solid form and may be aqueous, non-aqueous based or combination thereof and may be in the form of a lotion, cream, ointment, solution, foam, gel, solid stick, aerosol, bar, liquid, paste, powder, roll-on, sheet, spray, stick, tablet, suspension, emulsion, microemulsions, emusifiable concentrates, or the like. The formulation may be designed to be slowly released from a patch or be sprayed from a canister, a delivery apparatus or any suitable delivery system.

The carrier is a, topically cosmetic carrier, and pharmaceutically acceptable carriers are described herein.

In certain embodiments, the consumer care product described herein includes a composition comprising an insect or acarid repellant described herein and a delivery vehicle to deliver the composition to a selected location. The delivery vehicle can contain water, an aerosol, a cream, a gel, a lotion, an oil, a spray, a soap, a detergent, a particulate or a substrate. In other embodiments, the delivery vehicle can be a substrate that is a paper, a cloth or a woven or nonwoven material. In one embodiment, the substrate is a nonwoven material that is a flexible sheet containing fibers that are adhesively or thermally bonded. The fibers can be selected from among cellulose ester, cotton, hemp, jute, linen, ramie, rayon, polyamides, polyesters polyolefins, polypropylene, polyvinyl derivatives, silk, sisal and wool and combinations thereof.

In certain embodiments, the delivery vehicle can be a gel that contains a gelling agent that is selected from among agar, a carbomer, carboxyvinyl polymers, dibenzylidene alditols, carboxypolymethylene, collagen, dextrin fatty acid esters, gelatin, hydrogenated styrene/isoprene copolymers, 12-hydroxystearic acid, K-carrageenan, gellan gum, a lower hydroxy cellulose, pectin, polyacrylic acids, styrene-ethylene/propylene block copolymers, styrene-ethylene/butylene-styrene block copolymers, sucrose fatty acid esters and a wax and combinations thereof. In other embodiments, the delivery vehicle can be a fluid that when dispensed forms a gel in situ. The fluid can contain a gelling agent that selected from among agar, an alginate, a carbomer, carboxyvinyl polymers, dibenzylidene alditols, carboxypolymethylene, collagen, dextrin fatty acid esters, gelatin, hydrogenated styrene/isoprene copolymers, 12-hydroxystearic acid, K-carrageenan, gellan gum, a lower hydroxy cellulose, pectin, polyacrylic acids, styrene-ethylene/propylene block copolymers, styrene-ethylene/butylene-styrene block copolymers, sucrose fatty acid esters and a wax and combinations thereof.

In certain embodiments, the delivery vehicle can be or contain a particulate that is selected from among an alumina, amorphous silica, attapulgite, calcium carbonate, calcium phosphate, a clay, chalk, diatomaceous earths, fumed silica, a kaolin, kieselguhr, magnesium carbonate, microparticulate cellulose, montmorillonite, pyrophyllite, silicic acid, sodium bicarbonate, sodium carbonate, sodium phosphate, sodium pyrophosphate, talc, and vermiculite, and combinations thereof.

In certain embodiments, the insect or acarid repelled by a composition provided herein can be an insect selected from among Siphonaptera insects, such as cat flea (Ctenocephalides felis), dog flea (Ctenocephalides canis), oriental rat flea (Xenopsylla cheopis), human flea (Pulex irritans), chigoe (Tunga penetrans) and European rat flea (Nosopsyllus fasciatus); Anoplura insects, such as Head louse (Pediculus humanus capitis), crab louse (Pthirus pubis), short-nosed cattle louse (Haematopinus eurysternus), sheep louse (Dalmalinia ovis), hog louse (Haematopinus suis), long-nosed cattle louse (Linognathus vituli), cattle biting louse (Bovicola bovis), poultry shaft louse (Menopon gallinae), poultry body louse (Menacanthus stramineus), little blue cattle louse (Solenopotes capillatus), Haematopinus spp., Linognathus spp., Pediculus spp., Phtirus spp. and Solenopotes spp.; Acarina insects, such as bush tick (Haemaphysalis longicomis), Haemaphysalis flava, Dermacentor taiwanicus, American dog tick (Dermacentor variabilis), Ixodes ovatus, Ixodes persulcatus, black legged tick (Ixodes scapularis), lone star tick (Amblyomma americanum), Boophilus microplus, Rhipicephalus sanguineus, Ixodes holocyclus, western black legged tick (Ixodes pacificus), Dermacentor andersoni, Ambryomma maculatum, ear mite (Octodectes cynotis), Psoroptes spp., Chorioptes spp., Otodectes spp., Sarcoptes spp., Notoedres spp., Knemidocoptes spp., Sacroptes scabiei, Demodex spp., follicle mite (Demodex canis), northern fowl mite (Ornithonyssus sylviarum), poultry red mite (Dermanyssus gallinae), Trombicula spp., Leptotrombidium akamushi, Ornithodorus hermsi, Ornithodorus turicata, Ornithonyssus bacoti, Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Cytodites spp. and Laminosioptes spp.; Heteroptera insects, such as common bedbug (Cimex lectularius), tropical bedbug (Cimex hemipterus), Reduvius senilis, Triatoma spp. Rhodnius spp., Panstrongylus spp., and Arilus critatus; and Mallophage (Amblycera and Ischnocera) insects, such as Trimenopon spp., Menopon spp., Trinoton spp., Bovicola spp., Werneckiella spp., Lepikentron spp., Trichodectes spp. and Felicola spp.

In certain embodiments, the insect or acarid repelled by a composition provided herein can be an insect selected from among ants, bedbugs, carpet beetles, centipedes, chiggers, drain flies, dust mites, biting mites, earwigs, fleas, flies, gnats, hornets, lice, millipedes, mites, mosquitoes, roaches, scabies, silverfish, spiders, stinkbugs, termites, ticks, wasps, weevils and yellow jackets. The compositions comprising insect or acarid repellants provided herein, in certain embodiments, can be formulated for delivery of for at least 1 day, or at least 2 days, or at least 3 days, or at least 4 days, or at least 5 days, or at least 6 days, at least 7 days, or at least 8 days, or at least 9 days, or at least 10 days, or at least 11 days, or at least 12 days, at least 13 days, for at least 14 days, or at least 15 days, or at least 16 days, or at least 17 days, or at least 18 days, or at least 19 days, at least 20 days, or at least 21 days, or at least 22 days, or at least 23 days, or at least 24 days, or at least 25 days, at least 26 days, or at least 27 days, or at least 28 days, or at least 29 days, or at least 30 days, or at least 31 days, or at least 45 days, or at least 60 days or at least 75 days or at least 90 days.

Certain embodiments also include fabric treatment sheets containing a woven or nonwoven sheet or other substrate, including but not limited to cellulosic substrate, that is coated or impregnated with a fabric treatment composition and a composition comprising an insect or acarid repellant provided herein. The fabric treatment composition can be a detergent composition or a fabric softening composition. In some embodiments, the fabric treatment composition is a detergent composition that contains a detergent selected from among anionic surfactants, nonionic surfactants, zwitterionic surfactants, ampholytic surfactants and cationic surfactants and mixtures thereof. In other embodiments, the fabric treatment composition is a fabric softening composition that contains monomethyl trialkyl quaternaries, imidazolinium quaternaries, dimethyl alkyl benzyl quaternaries, dialkyl dimethyl quaternaries, methyl dialkoxy alkyl quaternaries, diamido amine-based quaternaries and dialkyl methyl benzyl quaternaries or (C8-C24) fatty acid amides or any combination thereof.

In certain embodiments of the method for repelling an insect or pest from a location, the location is the surface of the body of a human or animal. For example, the animal can be a companion animal or a farm animal. In other embodiments of the method, the composition is deployed by applying topically to an article of clothing of a human. In other embodiments of the method, the composition comprising an insect or acarid repellant provided herein is deployed by laundering an article of clothing of a human with a detergent or fabric softener or both that contains the composition. In yet another embodiment of the method, the composition is deployed by drying an article of clothing of a human with a fabric softener that contains the composition. The fabric softener can be provided as a dryer sheet or gel. In another embodiment of the method, the location is a surface that is skin, hair or fur and the composition is deployed by applying topically to the skin, hair or fur. The topically applied composition can be provided as an aerosol, a solution, an emulsion, an oil, a lotion, a soap, a spray, or a gel. In yet another embodiment of the method, the composition is provided to a surface that is skin, hair or fur in a form selected from among skin conditioners, hand/body/facial lotions, skin moisturizers, skin toners, skin sanitizers, skin cleansing compositions, skin soothing and lubricating compositions, sunscreen products, anti-aging products, tanning products, self-tanning products, after-sun products, masking products, anti-wrinkle products, hair conditioners, hair styling gels, hair anti-dandruff compositions, hair growth promoter compositions, hair lotions, hair tonics, rinses, conditioners, hair colorant compositions, hair anti-frizzing agent compositions, hair shining compositions, mousses, styling gels, hair pomade products and hair sprays, soaps, foaming bath products, hand/body/facial cleansers, astringent cleansers, anti-acne products, shampoos, body shampoos, synthetic detergent bars, shower gels and shampoos.

In certain embodiments, the insect or pest can be selected from among, but not limited to, ants, bedbugs, carpet beetles, centipedes, chiggers, drain flies, dust mites, earwigs, fleas, flies, gnats, hornets, lice, millipedes, mites, mosquitoes, roaches, scabies, silverfish, spiders, stinkbugs, termites, ticks, wasps, weevils and yellow jackets. For example, in the provided method, the insects or pests are ants that are selected from among Argentine ants, black ants, carpenter ants, fire ants, odorous house ants, pavement ants and pharaoh ants. In another embodiment, the insects or pests are lice that are selected from among head lice, body lice, pubic lice and nits thereof.

In certain embodiments, a composition comprising an insect or acarid repellant provided herein can be deployed by applying topically to an article of clothing of a human; or applying topically to skin or hair of a human; or applying topically to skin or fur of an animal. In some embodiments, the animal can be selected from among a bovine, canine, caprine, cervine, cricetine, feline, galline, equine, lapine, murine, musteline and ovine. For example, the animal is a companion animal. In other embodiments, the composition comprising an insect or acarid repellant provided herein can be deployed by laundering an article of clothing of a human with a detergent or fabric softener or both that contains the composition; or drying an article of clothing of a human with a fabric softener that contains the composition.

In some embodiments of a method for preventing skin injury due to biting insects or pests, the surface is skin or hair of a human and the composition comprising an insect or acarid repellant provided herein can be applied topically to the skin or hair. In an embodiment, the composition comprising an insect or acarid repellant provided herein can be provided as an aerosol, a solution, an emulsion, an oil, a lotion, a soap, a spray, or a gel. In yet other embodiment, the composition is provided in a form selected from among skin conditioners, hand/body/facial lotions, skin moisturizers, skin toners, skin sanitizers, skin cleansing compositions, skin soothing and lubricating compositions, sunscreen products, anti-aging products, tanning products, self-tanning products, after-sun products, masking products, anti-wrinkle products, hair conditioners, hair styling gels, hair anti-dandruff compositions, hair growth promoter compositions, hair lotions, hair tonics, rinses, conditioners, hair colorant compositions, hair anti-frizzing agent compositions, hair shining compositions, mousses, styling gels, hair pomade products and hair sprays, soaps, foaming bath products, hand/body/facial cleansers, astringent cleansers, anti-acne products, shampoos, body shampoos, synthetic detergent bars, shower gels and shampoos. The insect or pest can be selected from ants, bedbugs, chiggers, fleas, lice, mites, mosquitoes, roaches, scabies, and ticks.

In at least one embodiment, the disclosed invention includes a consumer care product prepared according to the process comprising (a) mixing an active ingredient and fatty acid; and (b) adding the mixture to a pharmaceutically suitable topical carrier.

In at least one embodiment, the disclosed invention includes a repellent pheromone such as necromone or an anthranilate ester, alone or in combination with aromatic oils. Preferably, the repellent pheromone is present in the amounts of 4% to 10% w/w. In another embodiment, the fatty acids of the presently claimed consumer care product include saturated and unsaturated fatty acids containing from 8 to 24 carbon atoms, with fatty acids containing from 13 to 21 carbon atoms being preferred. Illustrative of the fatty acids which may be employed are oleic acid, ricinoleic acid, linoleic acid palmitic acid and stearic acid; with steric and oleic acids being particularly preferred.

In one embodiment, the present invention concerns a topical skin care composition that contains water, a surfactant, emulsifying agent, a skin conditioning agent or an emollient, a humectant, silicon, an anti-inflammatory, a sun blocking agent, a topical anesthetic, vitamins, an antipruritic, an antibiotic, antifungal, an antiseptic, a vasoconstrictor, and other suitable topical or cosmetic excipients such as a pH modifier, a thickener, an anti-oxidant, a preservative and the like.

In certain embodiments, the composition further contains a dispersing agent that is selected from among, but not limited to, a surfactant, polyvinyl pyrrolidone, polyoxyethylated castor oil, a polyoxyethylene sorbitan ester, alkylnaphthalene sulfonate, alkylbenzenesulfonate, polyoxyethylene, polycarboxylate, lignin sulfonate, sodium silicate, potassium silicate, methylcellulose, carboxymethyl cellulose, hydroxypropylcellulose, hydroxypropyl-methylcellulose, gum arabic, a polyacrylate, and an acrylic/maleic copolymers and combinations thereof.

In at least one embodiment, the composition contains a surfactant in amounts of 0 to 40% w/w, or an emulsifying agent including, but not limited to, such compounds as anionics, cationics, non-ionic, amphoteric, zwitteronics, and combinations thereof. Exemplary of such surfactants and/or emulsifiers suitable for use with the present disclosure include, but are not limited to, esters, amides, polysaccharide ethers, polyglycosides, fatty acids, fatty alcohols, amine oxides, water-soluble cellulose derivatives, alkyl sulfonates, ethoxylated alkyl phenols, alkanolamides, betaines, zwitterionic surfactants, carboxylated alcohols, carboxylic acids, ethoxylated alcohols, preferably ethoxylated C₁₂-C₁₅ alcohols (ex. Rhodasurf^{®} L-9 brand); nonionic surfactants such as polysorbate 20, polysorbate 80, alkoxylated alcohol, a dialkoxylated alcohol, an alkoxylated dialkylphenol, an alkylpolyglycoside, an alkoxylated alkylphenol, an alkoxylated glycol, an alkoxylated mercaptan, an alkylamine salt, an alkyl quaternary amine salt, a glyceryl or polyglyceryl ester of a natural fatty acid, an alkoxylated glycol ester, an alkoxylated fatty acid, an alkoxylated alkanolamide, a polyalkoxylated silicone and an N-alkyl pyrrolidone and combinations thereof, anionic surfactants such as DEA phosphate, alkyl sulfonate surfactants, a linear alkylbenzene sulfonic acid, a branched alkylbenzene sulfonic acid a C12 to C18 alkylsulfate, C12-C18 alkyl alkoxy sulfate, C12-C18 alkyl methyl ester sulfonate and combinations thereof, cationic surfactants such as behentrimonium methosulfate, alkylamine, an alkyl diamine, an alkyl polyamine, a mono- or diquaternary ammonium salt, a monoalkoxylated amine, a dialkoxylated amine, a monoalkoxylated quaternary ammonium salt, a dialkoxylated quaternary ammonium salt, an etheramine, an amine oxide, an alkoxylated amine oxide and a fatty imidazoline and the like and derivatives thereof.

In at least one embodiment, the formulation is in the form of a personal care composition, such as a foaming composition that contains single emulsifier or a combination of two or more emulsifiers. In at least one embodiment, the formulation contains both an aqueous and an organic phase, wherein the organic phase contains a surfactant and an organic diluent. In one embodiment, the organic phase may comprise the active ingredient, isopropyl myristate and ethoxylated castor oil or the like (ex. Agnique^{®}BP-4-3101 brand).

The dispersing agent can be present in the provided compositions in an amount of at or about 0.002% to at or about 50% by weight of the composition. In some examples, the dispersing agent is present in an amount of at or about 0.025% to at or about 25% by weight of the composition. In other examples, the dispersing agent is present in an amount of at or about 0.01% to at or about 15% by weight of the composition. In yet other examples, the dispersing agent is present in an amount of at or about 0.05% to at or about 10% by weight of the composition, for example, in an amount at or about at least 0.05%, 0.06%, 0.07%, 008%, 0.09%, 0.1%, 0.15%, 0.2%, 0.25%, 0.3%, 0.35%, 0.3%, 0.35%, 0.5%, 0.55%, 0.6%, 0.65%, 0.7%, 0.75%, 0.8%, 0.85%, 0.9%, 0.95%, 1%, 1.05%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2%, 2.05%, 2.1%, 2.15%, 2.2%, 2.25%, 2.3%, 2.35%, 2.4%, 2.45%, 2.5%, 2.55%, 2.6%, 2.65%, 2.7%, 2.75%, 2.8%, 2.85%, 2.9%, 2.95%, 3%, 3.05%, 3.1%, 3.15%, 3.2%, 3.25%, 3.3%, 3.35%, 3.4%, 3.45%, 3.5%, 3.55%, 3.6%, 3.65%, 3.7%, 3.75%, 3.8%, 3.85%, 3.9%, 3.95%, 4%, 4.05%, 4.1%, 4.15%, 4.2%, 4.25%, 4.3%, 4.35%, 4.4%, 4.45%, 4.5%, 4.55%, 4.6%, 4.65%, 4.7%, 4.75%, 4.8%, 4.85%, 4.9%, 4.95%, 5%, 5.05%, 5.1%, 5.15%, 5.2%, 5.25%, 5.3%, 5.35%, 5.4%, 5.45%, 5.5%, 5.55%, 5.6%, 5.65%, 5.7%, 5.75%, 5.8%, 5.85%, 5.9%, 5.95%, 6%, 6.05%, 6.1%, 6.15%, 6.2%, 6.25%, 6.3%, 6.35%, 6.4%, 6.45%, 6.5%, 6.55%, 6.6%, 6.65%, 6.7%, 6.75%, 6.8%, 6.85%, 6.9%, 6.95%, 7%, 7.05%, 7.1%, 7.15%, 7.2%, 7.25%, 7.3%, 7.35%, 7.4%, 7.45%, 7.5%, 7.55%, 7.6%, 7.65%, 7.7%, 7.75%, 7.8%, 7.85%, 7.9%, 7.95%, 8%, 8.05%, 8.1%, 8.15%, 8.2%, 8.25%, 8.3%, 8.35%, 8.4%, 8.45%, 8.5%, 8.55%, 8.6%, 8.65%, 8.7%, 8.75%, 8.8%, 8.85%, 8.9%, 8.95%, 9%, 9.05%, 9.1%, 9.15%, 9.2%, 9.25%, 9.3%, 9.35%, 9.4%, 9.45%, 9.5%, 9.55%, 9.6%, 9.65%, 9.7%, 9.75%, 9.8%, 9.85%, 9.9%, 9.95% and 10% by weight of the composition.

In another embodiment, the composition may contain one or more thickening agents. In one embodiment, a thickening agent is present at a level of from about 0 to about 40 %, preferably from about 0.1% to about 30 %, and more preferably from about 0.25% to about 15%, by weight of the composition. Non-limiting classes of thickening agents include those selected from the following:
carboxylic acid polymers useful such as carbomers, crosslinked acrylic acid with allyl ethers of sucrose or pentaerythritol, generally available as the Carbopol series from B.F. Goodrich. Other thickening agent include crosslinked polyacrylate polymers, polyacrylamide polymers, polysaccharides or gelling agents such as cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Other thickening agents include gums such as acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluronic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboxymethyl dextran, sodium carrageenan, tragacanth gum, xanthan gum (Kelzan^{®}), and mixtures thereof.

In at least one embodiment the topical composition may further contain one or more topical anesthetics (or a plurality of topical anesthetics). Preferably, the topical anesthetic is lidocaine. In other embodiments, in lieu of, or in addition to, lidocaine, one or more of the following may be used: benzocaine, butamben, dibucaine, oxybuprocaine, pramoxine, proparacaine, proxymetacaine, tetracaine, amethocaine, propoxycaine, mepivacaine, bupivacaine, and the like, and mixtures thereof and equivalents.

In at least another embodiment, the anti-inflammatory agent can be a nonsteroidal such as diclofenac, salicylate naproxen, ketorolac and the like or a corticosteroid a such as hydrocortisone, prednisone, cortisol; dexamethasone, alcometasone; hydrocortisone 21-acetate; hydrocortisone 17-valerate; hydrocortisone 17-butyrate; bethamethasone 17,21-dipropionate; descinolone acetonide; bethamethasone valerate; prednisolone; fluorometholone; flucloronide; cortisone acetate; flunisolide acetate; triamcinolone acetonide; flucinonide; desconide; and the like; and mixtures thereof; and equivalents thereof. It is to be understood that any anti-inflammatory agent and/or topical corticosteroid may be used in the composition of the present invention. In other embodiments, the anti-inflammatory agent/topical corticosteroid is omitted.

In yet another embodiment, the composition comprises one or more antifungal agents. Preferably, the anti-fungal agent is nystatin; lotrimazole; fluconazole; ketoconazole; itraconazole; lotrimin; canesten; mycosporin; mycelex; clotrimazol; mykosporin; empecid; chlotrimazole, and the like, and mixtures thereof and equivalents thereof.

In yet another embodiment, the composition may further comprise a sun block or UV light blocker such as para-aminobenzoic acid (PABA), PABA esters (glyceryl PABA, amyldimethyl PABA and octyldimethyl PABA), butyl PABA, ethyl PABA, ethyl dihydroxypropyl PABA, benzophenones (oxybenzone, sulisobenzone, benzophenone, and benzophenone-1 through 12), avobenzone, cinnamates (and octyl methoxycinnamate, isoamyl p-methoxycinnamate, octylmethoxy cinnamate, cinoxate, diisopropyl methyl cinnamate, DEA-methoxycinnamate, ethyl diisopropylcinnamate, glyceryl octanoate dimethoxycinnamate and ethyl methoxycinnamate), cinnamate esters, salicylates (homomethyl salicylate, benzyl salicylate, glycol salicylate, isopropylbenzyl salicylate, etc.), trolamine salicylate, anthranilates, ethyl urocanate, homosalate, dibenzoylmethane derivatives (e.g., avobenzone), octocrylene, methylbenzylidene camphor, etc. and the brands Tinosorb M^{™}, Tinosorb S^{™}, Tinosorb A2B^{™}, Neo Helipan^{™}, Mexoryl XL^{™}, Uvinul T^{™}, Parsol SLX^{™}. Non-limiting examples of physical sunblocks include, kaolin, talc, petrolatum and metal oxides (e.g., titanium dioxide and zinc oxide or the like. Additional examples of skin conditioning agents, UV light blockers, thickeners, antioxidants, and pharmaceutically and cosmetically acceptable ingredients may be found in such texts as McCutcheons Emulsifiers and Detergents as well as (Vol 2) Functional Materials, both the North American Editions and the International Editions, published yearly by McCutcheon Publications,.

In yet another embodiment, the topical composition can further contain a skin conditioning agent or an emollient including a plant and/or a herbal component to make the external layers of the skin softer and more pliable. The emollient may be aloe vera extract, rose oil (i.e., the essential oils extracted from various types of rose), arnica extract, chamomile extract; chamomile flower extract; cetearyl alcohol; isopropyl myristate; triglyceride; myristic acid; palmitic acid; PEG-60 hydrogenated castor oil; glceryl linoleate; cyclomethicone; dimethicone; decyl oleate; stearic acid; lanolin; plant oils; shea butter; cocoa butter; algae extract; avocado oil; seed oil; buckthorn; calendula; camellia oil; capsaicin; castor oil; chamomile oil; lemongrass extract; lime oil; marula oil; olive oil; panthenol; corn oil; flaxseed oil; ginseng extract; haxel oil; pomegranate seed oil; rice bran oil; ginger; and the like, and mixtures thereof. In another embodiment, the topical composition may contain a vitamin such as vitamin A, B, C, E, beta-carotene; vitamin B complexes; vitamin B12, an antioxidant, a mineral such as sodium, potassium, calcium or the like. In other embodiments, the vitamin or minerals may be omitted.

Non-limiting examples of antioxidants that can be used with the compositions of the present invention include acetyl cysteine, ascorbic acid polypeptide, ascorbyl dipalmitate, ascorbyl methylsilanol pectinate, ascorbyl palmitate, ascorbyl stearate, BHA, BHT, t-butyl hydroquinone, cysteine, cysteine HCI, diamylhydroquinone, di-t-butylhydroquinone, dicetyl thiodipropionate, dioleyl tocopheryl methylsilanol, disodium ascorbyl sulfate, distearyl thiodipropionate, ditridecyl thiodipropionate, dodecyl gallate, erythorbic acid, esters of ascorbic acid, ethyl ferulate, ferulic acid, gallic acid esters, hydroquinone, isooctyl thioglycolate, kojic acid, magnesium ascorbate, magnesium ascorbyl phosphate, methylsilanol ascorbate, natural botanical anti-oxidants such as green tea or grape seed extracts, nordihydroguaiaretic acid, octyl gallate, phenylthioglycolic acid, potassium ascorbyl tocopheryl phosphate, potassium sulfite, propyl gallate, quinones, rosmarinic acid, sodium ascorbate, sodium bisulfite, sodium erythorbate, sodium metabisulfite, sodium sulfite, superoxide dismutase, sodium thioglycolate, sorbityl furfural, thiodiglycol, thiodiglycolamide, thiodiglycolic acid, thioglycolic acid, thiolactic acid, thiosalicylic acid, tocophereth-5, tocophereth-10, tocophereth-12, tocophereth-18, tocophereth-50, tocopherol, tocophersolan, tocopheryl acetate, tocopheryl linoleate, tocopheryl nicotinate, tocopheryl succinate, and tris(nonylphenyl) phosphite

Examples of preservatives include quaternary ammonium preservatives such as polyquaternium-1 and benzalkonium halides (e.g., benzalkonium chloride ("BAC") and benzalkonium bromide), parabens (e.g., methylparabens and propylparabens), phenoxyethanol, benzyl alcohol, chlorobutanol, phenol, sorbic acid, thimerosal, an azole, benzisothiazolin-3-one, benzalkonium quaternary compounds, benzyl alcohol, borates, 2-bromo-2-nitro-propane-1,3-diol, butylparaben, 5-chloro-2-methyl-4-isothiazolin-3-one, chlorphenesin, chlor-oxylenol, diazolidinyl urea, a dimethyl-benzylalkyl-ammonium chloride, ethyl paraben, formaldehyde, glutaraldehyde, halogenated salicylanilides, hexachlorophene, isobutyl-paraben, isothiazolin-3-one, 2-methyl-4-isothiazoline-3-one, methylparaben, mono-chloracetamide, neomycin sulfate, o-phenylphenol and salts thereof, phenoxyethanol, propionic acid and salts thereof, propylparaben, sodium benzoate, sorbic acid and salts thereof, tebuconazole and triazoles, or combinations thereof.

In certain embodiments, the compositions provided herein can further contain a colorant that is selected from among, but not limited to, a dye or pigment. In one example, the colorant is present in an amount at or about 0.0001% to at or about 1% by weight of the composition. In another example, the colorant is present in an amount at or about 0.0005% to at or about 0.5% by weight of the composition.

The compositions for killing or repelling pests provided herein are formulated as a personal care or cosmetic composition. In some applications, the personal care or cosmetic composition is formulated as a product selected from among insect repellents, skin care products, hair care products, and cleansing products. In some examples, the personal care or cosmetic composition is a skin care product that is selected from among skin conditioners, hand/body/facial lotions, skin moisturizers, skin toners, skin sanitizers, skin cleansing compositions, skin soothing and lubricating compositions, sunscreen products, anti-aging products, tanning products, self-tanning products, after-sun products, masking products and anti-wrinkle products. In other examples, the personal care or cosmetic composition is a hair care product that is selected from among hair conditioners, hair styling gels, hair anti-dandruff compositions, hair growth promoter compositions, hair lotions, hair tonics, rinses, conditioners, hair colorant compositions, hair anti-frizzing agent compositions, hair shining compositions, mousses, styling gels, hair pomade products and hair sprays. In other examples, the personal care or cosmetic composition is a cleansing product that is selected from among soaps, foaming bath products, hand/body/facial cleansers, astringent cleansers, anti-acne products, shampoos, body shampoos, synthetic detergent bars, shower gels and shampoos.

In at least one embodiment, the topical composition can contain a repellent component from 0.01 % to about 99% by weight. In at least one embodiment, the repellent component can range from 0.01 to 25 wt %. In one preferred embodiment, the repellant is methyl anthranilate (also may be referred herein as MA ) in ranges of about 2 to 15 wt %. In a more preferred embodiment, the amount of methyl anthranilate is below 8% and can be applied to a subject's skin in an amount up to about 5000 mg/kg.

In at least one embodiment, the topical formulation contains an organic and an aqueous phase. In one embodiment, the organic phase contains the repellant and optionally a surfactant or an emulsifying agent in combination with a fatty acid. In a preferred embodiment, the aqueous phase contains water, glycerin, lecithin, a C₁-C₆ alcohol or any combinations thereof. In an alternative embodiment, the aqueous phase may contain a thickening agent such as xanthan gum. In one embodiment, the aqueous phase may contain water in an amount ranging from about 5 % to about 50 %, preferably in the ranges of 40% to 45% and xanthan gum in an amount of 0 % to about 40%, preferably in the range of 20% to 30%, all % in % w/w.

In at least one embodiment, the topical formulation is in the form of an oil in water emulsion containing MA, oleic acid, steric acid, ethoxylated alcohol (e.x. Rhodasur^{®} L-9 brand), glycerin and water. In at least one preferred embodiment, the MA is in the ranges of about 2% to about 8 %, preferably 3.35% to 6.7 %. In another embodiment, oleic acid is in the ranges of about 0.5 to 3 %, preferably 0.065% to 1.3 %. In another embodiment, steric acid can be in the ranges of about 1 to 10 wt%, preferably in the ranges of 1.5 to 4.0 t%, all % are % w/w.

In at least another embodiment, the topical formulation contains diethylhexyl adipate, homosalate, octocrylene, octisalate, avobenzone, bemotrizinol, cyclopentasiloxane, cyclohexasiloxane, necromone and ethanol. In yet another embodiment, the formulation is in the form of a lotion containing water and lecithin in the aqueous phase, while containing oleic acid, methyl anthranilate and Caproyl 90^{™} in the organic phase. In yet another embodiment, the topical formulation is in the form of a body spray containing C₁₂-C₁₅ alkyl benzoate, triethylhexyl citrate, isododecane, necromone, polyamide-3 and denatured alcohol.

In at least one embodiment, the consumer care product comprises MA in an amount ranging from about 1% to about 10%; about 3% to about 7.5%; 3.35% to about 6.7%. In another embodiment, oleic acid (also refered herein as "OA") is in an amount ranging from about 0.1% to about 2.5%, preferably about 0.45% to about 1.3%; steric acid in an amount ranging from about 1% to about 8%, preferably about 2.0% to about 4.0 %; an ethoxylated C₁₂-C₁₅ alcohol in an amount ranging from about 1.5% to about 12%; preferably about 3.0% to about 6%, glycerin in amount of about 2.5 to about 15%; preferably about 5% and a solvent such as water in an amount of 25% to 85%, preferably about 75%, all % are % w/w.

Another aspect of the present invention is directed to a non-therapeutic method of repelling insect and/or acarid pests employing the methyl anthranilate /fatty acid compositions described above. Such compositions provide effective repellent activity against a number of insect orders including Hymenoptera, including pavement ant, carpenter ant, Argentine ant, little fire ant, ghost ant, cold tolerant ant, wood ant, Asian needle ant, odorus ant, rover ant, fire ant, bigheaded ant, honeybee, carpenter bee, bumble bee, small carpenter bee, European paper wasp, German yellowjacket and bald-faced hornet; Blattodea including German roach, Eastern subterranean termite; Hemiptera including bed bugs and brown marmorated stink bugs; Diptera including Aedes and Anopheles mosquitoes; Coleoptera including corn rootworm, sawtoothed beetle and confused flour beetle; Siphonaptera including cat flea and Lepidoptra including tobacco budworm. Repellent activity has also been demonstrated against brown dog ticks.

In at least another embodiment, the present invention is directed to a non-therapeutic method of repelling an insect from a subject comprising topically administering to a subject in need thereof a insect or acarid repellant composition as defined herein wherein the subject is a human, dogs, other domesticated animals or live cattle.

In another embodiment, the present topical compositions can be directly applied to the skin or a user's apparel and clothing. In yet another embodiment, the insect orders including Hymenoptera, including pavement ant, carpenter ant, Argentine ant, little fire ant, ghost ant, cold tolerant ant, wood ant, Asian needle ant, odorus ant, rover ant, fire ant, bigheaded ant, honeybee, carpenter bee, bumble bee, small carpenter bee, European paper wasp, German yellowjacket and bald-faced hornet; Blattodea including German roach, Eastern subterranean termite; Hemiptera including bed bugs and brown marmorated stink bugs; Diptera including Aedes and Anopheles mosquitoes; Coleoptera including corn rootworm, sawtoothed beetle and confused flour beetle; Siphonaptera including cat flea and Lepidoptra including tobacco budworm and brown dog ticks.

Preferred embodiments of the invention include: on-skin formulations containing a mixture of oleaic acid and methyl anthranilate, in the various proportions recited herein; household products such as sprays containing a mixture of oleaic acid and methyl anthranilate, in the various proportions recited herein; and household products such as clothing treatments containing a mixture of oleaic acid and methyl anthranilate, in the various proportions recited herein.

All lists used to describe the invention disclosed herein are open ended and contain other items, unless specifically stated otherwise.

### Formulation Examples

### Example 1

An esthetically pleasing and cosmetically acceptable skin care Formulation J was prepared in a lotion form in accordance to the following method.

Initially, an oil phase was prepared by combining and stirring the ingredients below heated to 60°C (beyond the melting point of stearic acid):

| | %w/w |
|---|---|
| Methyl Anthranilate (MA) | 6.7 |
| Oleic acid (OA) | 1.3 |
| Stearic acid | 2.0 |
| Rhodasurf L-9 | 3.0 |

The aqueous phase was then prepared by mixing the ingredients below heated to 60°C. Rhodasurf L-9 is the Rhodia brand of ethoxylated C12-C15 alcohols. Other vendor surfactants are also acceptable

| %w/w | | |
|---|---|---|
| | Water | 75.0 |
| | Glycerin | 5.0 |

Once both phases were prepared and heated to 60°C, they were combined with shear until emulsified lotion was formed. Milder stirring was commenced and the product was allowed to cool to 25°C. The lotion formulation formed was stable at 25° C.

### Example 2

Formulas K and L were prepared according to the following recipe:

| Ingredient | Formulation K | Formulation L |
|---|---|---|
| Aqueous phase: | | |
| Water | 50 | 50 |
| Lecithin | 6 | 6 |
| Organic Phase: | | |
| Oleic acid (OA) | 1.6 | 0.8 |
| Methyl anthranilate (MA) | 6.4 | 3.2 |
| Caproyl 90 | q.s | q.s |

These formulas were prepared by separately formulating the aqueous and oil phases. Aqueous phase was prepared by dissolving lecithin into water and heating to 80°C. Subsequently the organic phase was prepared by dissolving the OA and MA into Caproyl 90^{™} (propylene glycol monocaprylate type II NF) and heating to 80°C. Under high shear the organic phase was added to the aqueous phase and high shear was continued until particle size reaches 3 microns. Next, heating was discontinued and mild stirring commenced and continued until the lotion reached 25°C, at which time the product was packaged and tested for efficacy against biting pests.

The preliminary toxicity tests indicated favorable ophthalmic, dermatologic and oral toxicity profile. The efficacy of the lotion formulation was assessed and the results indicated favorable are depicted below:

**Table 1**

| Formula | % efficacy |
|---|---|
| 4% + 20% Isopropyl myristate ("IPM") 4% = 0.8% oleic acid + 3.2% methyl anthranilate (MA) | 99.2 |
| 8% + 20% IPM 8% = 1.6% oleic acid + 6.4% MA | 99.7 |
| 4% + 10% IPM^{∗} 4% = 0.8% oleic acid + 3.2%MA | 99.5 |
| 8% + 10% IPM 8% = 1.6% oleic acid + 6.4% MA | 99.8 |
| 8% Lotion (Formulation J) | 99.7 |
| 4% no IPM 4% = 0.8% oleic acid + 3.2% methyl anthranilate | 87.7 |

The lotions were superior in both efficacy and toxicity profile as compared to 20% Deet.

### Example 3

Additional skin care Formulations M, N, O in the form of body spray containing repellent mixtures were prepared containing the ingredients listed below:

These formulas may contain sunscreen, to further protect subjects not only against insect attack but also the harmful solar rays. Preferably, these additional formulas are clear, low viscosity body sprays.

In preparing Formulation M, phases A, B and C were prepared separately. Phase A was heated to 80°C until all ingredients are dissolved. Phase A was then cooled down to room temperature and mixed with respectively phase B and then phase C while stirring. These examples further define formulations of Necromone Manufacturing Use Product (or MUP), in all cases defined for the examples as 1 part oleic acid to 4 parts methyl anthranilate.

| ***Phase A*** | |
|---|---|
| | **%*w*/*w*** |
| ***Dub DOA (Stearinerie Dubois Fils) (Diethylhexyl adipate)*** | ***13.00*** |
| ***Eusolex HMS (Merck) (Homosalate)*** | ***15.00*** |
| ***Neo Heliopan 303 (Symrise) (Octocrylene)*** | ***10.00*** |
| ***Neo Heliopan OS (Symrise) (Octisalate)*** | ***5.00*** |
| ***Parsol 1789 (DSM) (Avobenzone)*** | ***3.00*** |
| ***Tinosorb S (Ciba) (Bemotrizinol)*** | ***2.00*** |

| ***Phase B*** | |
|---|---|
| ***Dow Corning 345 fluid (Dow Corning) (Cyclopentasiloxane (and) cyclohexasiloxane)*** | ***5.00*** |

| ***Phase C*** | |
|---|---|
| ***Necromone MUP*** | ***8.00*** |
| ***Ethanol 96% (Merck)*** | ***q.s. to 100%*** |

In preparing Formulation N, phases A and B were prepared separately. Phase A was then heated to 80°C until all ingredients were dissolved. Phase A was then cooled down to room temperature and mixed with phase B while stirring.

| Phase A | |
|---|---|
| Finsolv TN (Innospec) (C12-15 alkyl benzoate) | 65.20 |
| Nikkol TOC (Nikko Chemical) (Triethylhexyl citrate) | 7.00 |
| Permethyl 99A (Presperse) (Isododecane) | 17.00 |
| Necromone MUP | 8.00 |

| Phase B | |
|---|---|
| Sylvasol 80-E70 (Arizona Chemical) (Alcohol denat. and polyamide-3) | 6.80 |

In Preparing Formulation O, phases A and B were prepared separately. Phase A was then heated to 80°C until all ingredients were dissolved. Phase A was then cooled down to room temperature and mixed with phase B while stirring.

| Phase A | |
|---|---|
| DI water | 42 |
| 2% Kelzan (xanthan gum) | 14 |
| Necromone MUP | 8.00 |

| Phase B | |
|---|---|
| Sylvasol 80-E70 (Arizona Chemical) (Alcohol denat. and polyamide-3) | 6.80 |

### Example 4

Female adult *Aedes aegypti* mosquitoes were obtained from i2LResearch USA, Inc.'s in-house colony. 250 mosquitoes were used per replicate (5 replicates per test). Adult mosquitoes were 5-12 days old and were deprived of sucrose solution for approximately 18 hours prior to testing.

The following test substances were assessed:

**Table 2**

| | **Formulation** | **Active ingredients** |
|---|---|---|
| 1 | Formulation P | 20% IPM, 4% Necromone MUP in EtOH |
| 2 | Formulation Q | 20% IPM, 8% Necromone MUP in EtOH |
| 3 | Formulation R | 10% IPM, 4% Necromone MUP in EtOH |
| 4 | Formulation S | 10% IPM, 8% Necromone MUP in EtOH |
| 5 | Formulation T | 8% Necromone MUP |
| 6 | Formulation U | 4% Necromone MUP in EtOH |

The test substances were applied directly to prepared collagen membranes by pipetting 25µL of the appropriate substance onto a membrane and spreading it evenly with the tip of the pipette.

*Exposure container.* A 30.5 cm x 30.5 cm x 30.5 cm rigid plastic frame supported by four, 4-cm high legs, with a sleeved entry on one side and a sliding door on the bottom.

*Membrane feeder.* Five wells (3 cm in diameter x 8 mm in depth) in line on a hollow plastic block (6 cm wide x 22 cm long x 3 cm deep), which fits through the sliding door in the bottom of the exposure container. Hoses attached to each side of the block circulate heated water that is pumped from a water bath. The sliding door in the bottom of the exposure container covers and uncovers the wells in the membrane feeder, allowing mosquitoes to access the wells.

*Test set-up.* The membrane feeder was connected to a heated water bath, and warm water passed through the feeder via a circulating pump so that the wells were warmed to 32-35°C (89-95 °F). Seventy-two (72) mg of ATP (disodium salt) were added to 26 mL of warmed citrated bovine blood, which was poured into the wells until they were completely full.

The collagen membranes were briefly dipped in tap water and blotted with a paper towel then placed flat on a metal tray covered in wax paper (using vacuum grease as a temporary adhesive around the edges of the membranes). The membranes were then treated as described above, and left to age for two hours on a heating pad at 93-96° F. An additional membrane was prepared in the same way except that it received no treatment, in order to be used as a negative control. After the two hour aging interval, the membranes were placed over each of the wells (with vacuum grease as adhesive), completely covering the blood. Care was taken to eliminate all air bubbles from between the membrane and the surface of the blood.

*Exposure to mosquitoes.* The mosquitoes were released into the exposure container just prior to exposure to the membranes. The exposure container was then placed on the membrane feeder and the sliding door opened, allowing the mosquitoes to access the wells. The number of mosquitoes probing each membrane was recorded every two minutes for twenty minutes.

The above procedures were repeated until five replicates were completed. A new batch of 250 female mosquitoes and fresh blood were used for each replicate, and the wells were cleaned in between replicates. The position of the treatments was rotated for each replicate, so that each treatment was tested on each of the five wells.

A total of three tests were conducted, each assessing a combination of four of the formulations against an untreated membrane. Each formulation was assessed twice over the course of the three test days.

Statistical analyses. Percent repellency was calculated for each replicate using the total number of probes with the following formula: Repellency = ((C-T)/T) ^{∗} 100, where c = the total number of probes on the untreated well, and T = the total number of probes on the treated well.

Average percent repellency and standard error were then calculated across all five replicates.

**Table 3. Repellency of six experimental formulations exposed to adult female Aedes aegypti mosquitoes after being aged for 2 hours (percent repellency based on comparison to untreated).**

| Formulation | Average % repellency (SE) | | |
|---|---|---|---|
| | Test 1 | Test 2 | Test 3 |
| Formulation P | 99.2 (0.8) | - | 97.1 (2.9) |
| Formulation Q | 100 (0) | 99.6 (0.4) | - |
| Formulation R | 99.8 (0.2) | 98.8 (1.1) | - |
| Formulation S | - | 100 (0) | 99.2 (0.7) |
| Formulation T | - | 98.9 (1.1) | 99.7 (0.3) |
| Formulation U | 81.3 (10.5) | - | 95.6 (1.9) |

### Example 5

An in vitro laboratory trial was conducted to evaluate the repellency of three experimental formulations (Formulation J, Formulation K, and Formulation L), aged for two and/or six hours after application, against female mosquitoes. Testing was conducted over three test days: two days to assess the two-hour aging interval, and one day to assess the six-hour aging interval. Two positive controls (20% DEET and Off! Botanicals^{®} repellant) and an untreated (negative) control were also assessed on each test day for comparative purposes.

**Table 4**

| Formulation | Active ingredients |
|---|---|
| Off! Botanicals^{®} repellant | 10% p-menthane-3,8-diol |
| DEET in ethanol | 20% DEET |

Test substances were applied to prepared collagen membranes and aged for the appropriate time interval on a heating pad. The membranes were then placed over heated, blood-filled wells and exposed to mosquitoes. The number of mosquitoes probing each well was recorded at two minute intervals up to 20 minutes.

Results for Aedes aegypti: Treatments Formulation K and Formulation L each provided consistently high repellency across both time intervals (99.4-100% and 100%, respectively). Formulation J demonstrated significantly lower repellency than most other treatments at the two hour aging intervals (66.4-70.1%). Results are summarized in Table 5 below.

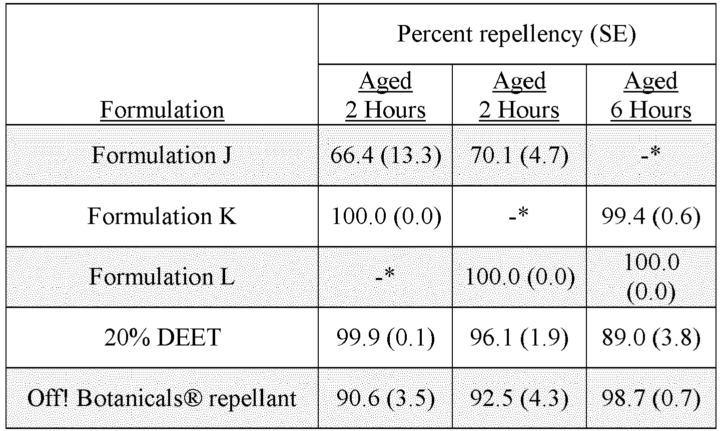

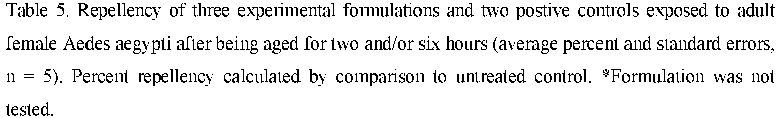

**Results for *Anopheles quadrimaculatus:*** Treatments Formulation K and Formulation L each provided satisfactory repellency across both time intervals (83.6-95.8% and 87.7-91.4%, respectively) and provided similar efficacy to 20% DEET at two hours and Off! Botanicals^{®} repellant at both ageing intervals. At six hours the experimental treatments provided superior efficacy compared to 20% DEET (75%). Results are summarized in Table 6 below.

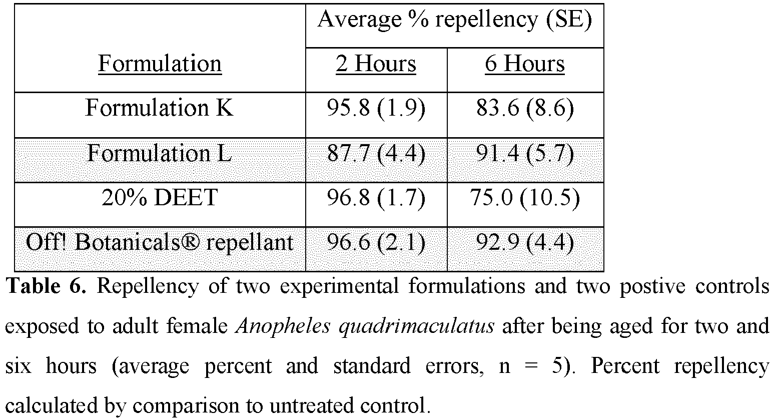

**Results for** *Culex quinquefasciatus:* Treatments Formulation K and Formulation L each provided consistently high repellency across both time intervals (96.9-100% and 99.5-100%, respectively) and provided similar efficacy to both 20% DEET and Off! Botanicals^{®} repellant. Results are summarized in Table 7 below.

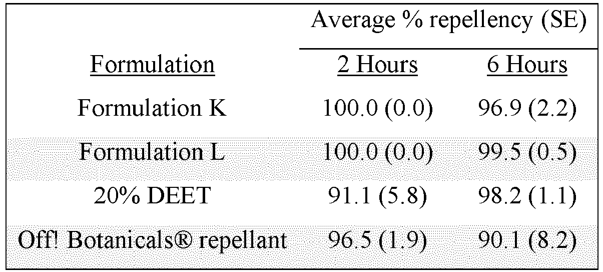

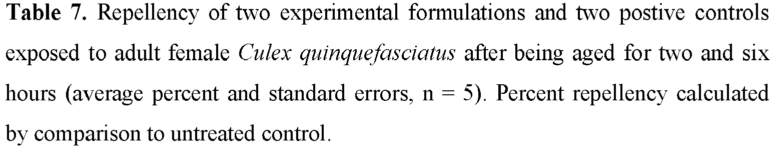

In conclusion, treatments Formulation K and Formulation L were effective at repelling mosquitoes even when aged for up to six hours.

## Claims

1. A consumer care product comprising an insect- or acarid-repellant composition comprising:
(a) methyl anthranilate;
(b) a fatty acid selected from the group consisting of oleic acid, ricinoleic acid, linoleic acid, palmitic acid, stearic acid and any mixtures thereof;
(c) optionally at least one additional active ingredient;
(d) a pharmaceutically suitable topical carrier; and
(e) an additional compound selected from the group consisting of a surfactant, emulsifying agent, a pH modifier, a thickening agent, an anti-oxidant, a preservative, a skin conditioning agent, an emollient, a humectant, a silicone moiety, an anti-inflammatory, a sun blocking agent, a topical anesthetic, vitamins, an antipruritic, an antibiotic, antifungal, an antiseptic, a vasoconstrictor, and any mixtures thereof; wherein the weight ratio of methyl anthranilate to the fatty acid of part (b) is in the range of 5:1 to 4:1.

2. The consumer care product of claim 1, wherein the fatty acid is oleic acid.

3. The consumer care product of claim 1, wherein the emulsifying agent of part (e) is selected from the group consisting of polysaccharide ethers, polyglycosides, fatty acids, fatty alcohols, amine oxides, water-soluble cellulose, alkyl sulfonates, ethoxylated alkyl phenols, alkanolamides, betaines, zwitterionic surfactants, carboxylated alcohols, carboxylic acids, ethoxylated C₁₂-C₁₅ alcohols, polysorbates, behentrimonium methosulfate, isopropyl myristate, ethoxylated castor oil and any mixtures thereof.

4. The consumer care product of claim 1, wherein the thickening agent is selected from the group consisting of carboxylic acid polymers, crosslinked acrylic acid with allyl ethers of sucrose, crosslinked polyacrylate polymers, polyacrylamide polymers, polysaccharides, gums and any mixtures thereof.

5. The consumer care product of claim 4, wherein the polysaccharides are selected from the group consisting of cellulose, carboxymethyl hydroxyethylcellulose, cellulose acetate propionate carboxylate, hydroxyethylcellulose, hydroxyethyl ethylcellulose, hydroxypropylcellulose, hydroxypropylmethylcellulose, methyl hydroxyethylcellulose, microcrystalline cellulose, sodium cellulose sulfate, chitosan and any mixtures thereof.

6. The consumer care product of claim 5, wherein the pharmaceutically suitable topical carrier of part (d) comprises water, glycerin, lecithin, a fatty acid or any combinations thereof.

7. A non-therapeutic method for repelling insects or acarids by applying the composition of claim 1 to the surface of a human or an animal.

## Patentansprüche

1. Ein Verbraucherpflegeprodukt, das eine Zusammensetzung zum Abwehren von Insekten oder Milben beinhaltet, beinhaltend:
(a) Methylanthranilat;
(b) eine Fettsäure, die aus der Gruppe ausgewählt ist, die aus Ölsäure, Ricinolsäure, Linolsäure, Palmitinsäure, Stearinsäure und beliebigen Mischungen davon besteht;
(c) optional mindestens einen zusätzlichen Wirkstoff;
(d) einen pharmazeutisch geeigneten topischen Träger; und
(e) eine zusätzliche Verbindung, die aus der Gruppe ausgewählt ist, die aus einem Tensid, einem Emulgiermittel, einem pH-Modifikator, einem Verdickungsmittel, einem Antioxidationsmittel, einem Konservierungsmittel, einem hautkonditionierenden Mittel, einem Erweichungsmittel, einem Feuchthaltemittel, einem Silikonanteil, einem Antiphlogistikum, einem Sonnenblockmittel, einem topischen Anästhetikum, Vitaminen, einem antipruriginösen Mittel, einem Antibiotikum, einem Antimykotikum, einem Antiseptikum, einem Vasokonstriktor und beliebigen Mischungen davon besteht;
wobei das Gewichtsverhältnis von Methylanthranilat zu der Fettsäure von Teil (b) im Bereich von 5 : 1 bis 4 : 1 liegt.

2. Verbraucherpflegeprodukt gemäß Anspruch 1, wobei die Fettsäure Ölsäure ist.

3. Verbraucherpflegeprodukt gemäß Anspruch 1, wobei das Emulgiermittel von Teil (e) aus der Gruppe ausgewählt ist, die aus Polysaccharidethern, Polyglykosiden, Fettsäuren, Fettalkoholen, Aminoxiden, wasserlöslicher Cellulose, Alkylsulfonaten, ethoxylierten Alkylphenolen, Alkanolamiden, Betainen, zwitterionischen Tensiden, carboxylierten Alkoholen, Carbonsäuren, ethoxylierten C₁₂-C₁₅-Alkoholen, Polysorbaten, Behentrimoniummethosulfat, Isopropylmyristat, ethoxyliertem Castoröl und beliebigen Mischungen davon besteht.

4. Verbraucherpflegeprodukt gemäß Anspruch 1, wobei das Verdickungsmittel aus der Gruppe ausgewählt ist, die aus Carbonsäurepolymeren, vernetzter Acrylsäure mit Allylethern von Saccharose, vernetzten Polyacrylatpolymeren, Polyacrylamidpolymeren, Polysacchariden, Gummi und beliebigen Mischungen davon besteht.

5. Verbraucherpflegeprodukt gemäß Anspruch 4, wobei die Polysaccharide aus der Gruppe ausgewählt sind, die aus Cellulose, Carboxymethylhydroxyethylcellulose, Celluloseacetatpropionatcarboxylat, Hydroxyethylcellulose, Hydroxyethylethylcellulose, Hydroxypropylcellulose, Hydroxypropylmethylcellulose, Methylhydroxyethylcellulose, mikrokristalliner Cellulose, Natriumcellulosesulfat, Chitosan und beliebigen Mischungen davon besteht.

6. Verbraucherpflegeprodukt gemäß Anspruch 5, wobei der pharmazeutisch geeignete topische Träger von Teil (d) Wasser, Glycerin, Lecithin, eine Fettsäure oder beliebige Kombinationen davon beinhaltet.

7. Ein nicht-therapeutisches Verfahren zum Abwehren von Insekten oder Milben durch das Anwenden der Zusammensetzung gemäß Anspruch 1 auf die Oberfläche eines Menschen oder eines Tiers.

## Revendications

1. Un produit de soin de consommation comprenant une composition répulsive contre les insectes ou les acariens comprenant :
(a) de l'anthranilate de méthyle ;
(b) un acide gras sélectionné dans le groupe constitué d'acide oléique, d'acide ricinoléique, d'acide linoléique, d'acide palmitique, d'acide stéarique et de tous mélanges de ceux-ci ;
(c) facultativement au moins un principe actif supplémentaire ;
(d) un support topique pharmaceutiquement adéquat ; et
(e) un composé additionnel sélectionné dans le groupe constitué d'un tensioactif, d'un agent émulsifiant, d'un modificateur de pH, d'un agent épaississant, d'un antioxydant, d'un conservateur, d'un agent conditionneur de la peau, d'un émollient, d'un humectant, d'un groupement silicone, d'un anti-inflammatoire, d'un agent antisolaire, d'un anesthésique topique, de vitamines, d'un antiprurigineux, d'un antibiotique, d'un antifongique, d'un antiseptique, d'un vasoconstricteur, et de tous mélanges de ceux-ci ;
où le rapport en poids de l'anthranilate de méthyle à l'acide gras de la partie (b) est compris dans la gamme allant de 5/1 à 4/1.

2. Le produit de soin de consommation de la revendication 1, où l'acide gras est l'acide oléique.

3. Le produit de soin de consommation de la revendication 1, où l'agent émulsifiant de la partie (e) est sélectionné dans le groupe constitué d'éthers de polysaccharide, de polyglycosides, d'acides gras, d'alcools gras, d'oxydes d'amine, de cellulose hydrosoluble, d'alkyl sulfonates, d'alkyl phénols éthoxylés, d'alcanolamides, de bétaïnes, de tensioactifs zwittérioniques, d'alcools carboxylés, d'acides carboxyliques, d'alcools en C₁₂-C₁₅ éthoxylés, de polysorbates, de méthosulfate de behentrimonium, de myristate d'isopropyle, d'huile de ricin éthoxylée et de tous mélanges de ceux-ci.

4. Le produit de soin de consommation de la revendication 1, où l'agent épaississant est sélectionné dans le groupe constitué de polymères d'acide carboxylique, d'acide acrylique réticulé avec des éthers allyliques de sucrose, de polymères de polyacrylate réticulés, de polymères de polyacrylamide, de polysaccharides, de gommes et de tous mélanges de ceux-ci.

5. Le produit de soin de consommation de la revendication 4, où les polysaccharides sont sélectionnés dans le groupe constitué de cellulose, de carboxyméthyl hydroxyéthylcellulose, d'acétate propionate carboxylate de cellulose, d'hydroxyéthylcellulose, d'hydroxyéthyl éthylcellulose, d'hydroxypropylcellulose, d'hydroxypropylméthylcellulose, de méthyl hydroxyéthylcellulose, de cellulose microcristalline, de sulfate sodique de cellulose, de chitosane et de tous mélanges de ceux-ci.

6. Le produit de soin de consommation de la revendication 5, où le support topique pharmaceutiquement adéquat de la partie (d) comprend de l'eau, de la glycérine, de la lécithine, un acide gras ou toutes combinaisons de ceux-ci.

7. Une méthode non-thérapeutique destinée à la répulsion d'insectes ou d'acariens par application de la composition de la revendication 1 sur la surface d'un être humain ou d'un animal.
